# EUROPÄISCHE PATENTSCHRIFT

(11) **EP 2 547 374 B1**
(45) Veröffentlichungstag und Bekanntmachung des Hinweises auf die Patenterteilung: **27.01.2016**
(21) Anmeldenummer: 11709642.0
(22) Anmeldetag: 14.03.2011
(51) Int. Cl.: A61M 1/00, A61M 1/16, B62B 5/00

(54) **BLUTBEHANDLUNGSGERÄT**
BLOOD TREATMENT DEVICE
APPAREIL DE TRAITEMENT DU SANG

(30) Priorität: 15.03.2010 DE 102010011464
(43) Veröffentlichungstag der Anmeldung: 23.01.2013
(73) Patentinhaber: Fresenius Medical Care Deutschland GmbH, 61352 Bad Homburg (DE)
(72) Erfinder: ÖRTER, Gökhan, 35789 Weilmünster (DE); OESTERREICH, Stefan, 61267 Neu-Anspach (DE)
(74) Vertreter: Herrmann, Uwe
(86) Internationale Anmeldenummer: PCT/EP2011/001260
(87) Internationale Veröffentlichungsnummer: WO 2011/113571

(56) Entgegenhaltungen:
- WO-A1-99/00154
- US-A1- 2004 064 080
- US-A1- 2008 230 450
- US-A1- 2008 272 564

## Beschreibung

Die vorliegende Erfindung betrifft ein Blutbehandlungsgerät, insbesondere ein Dialysegerät, mit Mitteln zur Durchführung einer Blutbehandlung, insbesondere einer Dialysebehandlung, und/oder ein Gerät mit Mitteln zur Befüllung und/oder zur Entleerung einer mobilen Vorrichtung mit einer bzw. von einer Behandlungsflüssigkeit, insbesondere einer Dialysierflüssigkeit.

Aus dem Stand der Technik sind Dialysegeräte bekannt, bei denen die Dialysierflüssigkeit nicht während einer Behandlung angesetzt wird, sondern bei denen die gesamte für eine Dialysebehandlung erforderliche Menge an Dialysierflüssigkeit vor der Behandlung in einem Tank bereitgestellt wird. Derartige Dialysegeräte werden auch als "Batch-Type"-Dialysegeräte bezeichnet.

Aus dem Stand der Technik gemäß der WO 2008/104367 A2 ist es des weiteren bekannt, diese Geräte mit einer mobilen Einheit zu verbinden, in der die Dialysierflüssigkeit zubereitet wird und von der die zubereitete Dialysierflüssigkeit in den Tank des Dialysegerätes eingefüllt wird.

Mobile Vorrichtungen für den Transport von Dialysierflüssigkeiten sind ferner aus der DE 103 13 965 B3 sowie aus der AU 40178/85 bekannt.

Ein System mit einem Behandlungsgerät und einer mobilen Vorrichtung mit einem Tank ist aus WO 99/00154 bekannt.

Der vorliegenden Erfindung liegt die Aufgabe zugrunde, ein Gerät der eingangs genannten Art dahingehend weiterzubilden, dass es auf einfache und zuverlässige Art und Weise mit einer mobilen Vorrichtung verbindbar ist, in der die Behandlungsflüssigkeit, insbesondere die Dialysierflüssigkeit vorliegt, bzw. die mit einer Behandlungsflüssigkeit zu befüllen ist.

Diese Aufgabe wird durch ein System mit den Merkmalen des Anspruchs 1 gelöst. Danach ist vorgesehen, dass das im System enthaltene Gerät wenigstens eine Aufnahme aufweist, die derart ausgebildet ist, dass in der Aufnahme eine mobile Vorrichtung aufnehmbar ist, wobei das Gerät Führungsmittel und/oder Fixierungsmittel aufweist, die derart ausgeführt sind, dass die mobile Vorrichtung mit Hilfe der Führungsmittel und/oder der Fixierungsmittel in die Aufnahme einführbar und/oder in der Aufnahme fixierbar ist. Der Begriff "Gerät" umfasst jedes beliebige Blutbehandlungsgerät sowie jedes beliebige Gerät, mittels dessen die genannte mobile Vorrichtung befüllbar und/oder entleerbar ist.

Es ist vorgesehen, dass die mobile Vorrichtung mit wenigstens einem Tank ausgeführt ist, in dem eine Behandlungsflüssigkeit zur Durchführung der Behandlung vorliegt oder aufnehmbar ist.

Die vorliegende Erfindung umfasst des weiteren eine mobile Vorrichtung mit wenigstens einem Tank, in dem sich eine Behandlungsflüssigkeit, insbesondere eine Dialysierflüssigkeit, befindet oder aufnehmbar ist, zur Versorgung eines Blutbehandlungsgerätes, insbesondere eines Dialysegerätes, mit der Behandlungsflüssigkeit während einer durch das Blutbehandlungsgerät durchgeführten Blutbehandlung, oder zur Verbindung mit einem Gerät mit Mitteln zur Befüllung und/oder Entleerung der mobilen Vorrichtung mit einer bzw. von einer Behandlungsflüssigkeit, insbesondere Dialysierflüssigkeit, wobei die mobile Vorrichtung Führungsmittel und/oder Fixierungsmittel aufweiset, die derart ausgeführt sind, dass die mobile Vorrichtung mit Hilfe der Führungsmittel und/oder der Fixierungsmittel in eine Aufnahme des Gerätes einführbar und/oder in einer Aufnahme des Gerätes fixierbar ist.

Für den Fall, dass die mobile Vorrichtung das gesamte zur Behandlung notwendige Volumen an Dialysierflüssigkeit trägt, kann das Eigengewicht der mobilen Vorrichtung einschließlich des Gewichtes der Dialysierflüssigkeit ohne weiteres bis zu 100 kg betragen. Denkbar ist es, dass die mobile Vorrichtung mittels Rollen manövrierbar ist, wobei dies den Vorteil hat, dass auf diese Weise die Handhabung der mobilen Vorrichtung trotz ihres hohen Gewichts entsprechend einfach ist und im Klinikalltag praktikabel umgesetzt werden kann.

Weiterhin ist es wichtig, dass die mobile Vorrichtung aus Gründen der Sicherheit und des Verletzungsrisikos stationär sicher aufgestellt werden kann und dass die mobile Vorrichtung weitgehend bewegungsfrei in der Befüll- bzw. Entleerungseinheit und der Behandlungsstation steht.

Vorzugsweise ist vorgesehen, dass in den Positionen, in denen die mobile Vorrichtung mit dem Gerät in Verbindung steht bzw. nach den Verfahren in die Aufnahme deren integraler Bestandteil bildet, fluidführende Leitungsverbindungen zwischen der mobilen Vorrichtung und dem Gerät bestehen, die durch mechanische Einflüsse wie Vibrationen, Schwankungen, etc. der mobilen Vorrichtung bzw. von deren Tank nicht unterbrochen werden dürfen. Ebenso ist es denkbar, dass zwischen der mobilen Vorrichtung und dem Gerät elektronische/elektrische Verbindungen bestehen, die nicht durch ungewollte Bewegungen der mobilen Vorrichtung unterbrochen werden dürfen.

Denkbar ist es grundsätzlich, dass das Gerät einen Teil der Standsicherheit selbst bildet, da es wie eine Stützvorrichtung wirken kann, wenn die mobile Vorrichtung eingeschoben ist. Auf diese Weise ist es möglich, ein seitliches Schwanken weitgehend durch diese Stützvorrichtungen zu verhindern.

Vorzugsweise ist des weiteren vorgesehen, dass Vorkehrungen gegen das Wegrollen der mobilen Vorrichtung aus ihrer in die Aufnahme eingeführten Position heraus getroffen werden. Denkbar ist es, arretierbare Rollen einzusetzen, die nach dem Einführen der mobilen Vorrichtung in die Aufnahme des Gerätes arretiert werden können.

Wird die Arretierung versehentlich nicht betätigt, besteht allerdings die Gefahr, dass die mobile Vorrichtung ungewollt aus ihrer vorgesehen Position wegbewegt wird oder wegrollt.

Um ein einfaches Einführen der mobilen Vorrichtung in die Aufnahme zu gewährleisten und/oder um die mobile Vorrichtung in der gewünschten Position zu halten sind Führungs- und/oder Fixierungsmittel an dem Gerät und/oder an der mobilen Vorrichtung vorgesehen.

Die Führungsmittel können beispielsweise als Führungszunge oder Schiene oder sonstige Führung ausgeführt sein. Diese Führungszunge bzw. Führungsmittel bietet eine gute Möglichkeit, die vergleichsweise schwere, gefüllte mobile Vorrichtung in das Blutbehandlungsgerät bzw. die Befüll- oder Entleereinheit einzuführen, ohne dass sich beim Einschieben die mobile Vorrichtung und das Gerät miteinander verhaken können und damit gegebenenfalls die Konstruktion schädigen können.

Erfindungsgemäß weisen die Führungsmittel und insbesondere die genannte Führungszunge, eine Nut oder sonstige Führungsbahn auf, in der oder auf der ein Gegenstück des jeweils anderen Teils, das heißt des Gerätes oder der mobilen Vorrichtung läuft.

Vorzugsweise befindet sich die Nut bzw. Führungsbahn auf der Oberseite der Führungszunge.

In weiterer Ausgestaltung der Erfindung ist vorgesehen, dass die Nut oder Führungsbahn wenigstens eine Vertiefung aufweist. Diese Vertiefung kann einen Bestandteil der Fixierungsmittel dahingehend bilden, dass ein Bestandteil der jeweils anderen Vorrichtung, das heißt entweder des Gerätes oder der mobilen Vorrichtung in die Vertiefung eingreift, wenn sich die mobile Vorrichtung in ihrer gewünschten, eingeführten Position in der Aufnahme befindet.

Die Vertiefung ist vorzugsweise an dem Nutende bzw. am Ende der Führungsbahn angeordnet.

In weiterer Ausgestaltung der Erfindung ist vorgesehen, dass die Nut wenigstens eine Einführöffnung aufweist, die eine größere Breite aufweist, als der sich an die Einführöffnung in Einführrichtung anschließende Teil der Nut oder Bahn. Auf diese Weise ist es möglich, Ungenauigkeiten während des Andockens der mobilen Vorrichtung durch den Nutzer zu kompensieren.

In weiterer Ausgestaltung der Erfindung ist vorgesehen, dass eine Einführrichtung besteht, in der die mobile Vorrichtung in die Aufnahme des Gerätes einführbar ist, wobei vorgesehen sein kann, dass sich die Führungszunge in Einführrichtung erstreckt. Denkbar ist es beispielsweise, dass das Blutbehandlungsgerät bzw. die Befülleinheit oder Entleerungseinheit eine offene Seite aufweist, in die die mobile Vorrichtung in einer Einführrichtung einführbar ist und dass sich die genannte Führungszunge in Einführrichtung erstreckt.

In weiterer Ausgestaltung der Erfindung ist vorgesehen, dass die Fixierungsmittel Mittel zur formschlüssigen Fixierung, insbesondere Rastmittel umfassen oder aus diesen bestehen.

Weiterhin ist vorgesehen, dass die Fixierungsmittel und/oder Führungsmittel wenigstens eine Kugelrolle bzw. Kugel oder Walze oder ein Gleitstück oder ein Kufenstück umfassen.

Denkbar ist es, ein Gleitstück beispielsweise aus Teflon zu arbeiten oder mit einer Teflon-Beschichtung zu versehen, um die Bedienkräfte herabzusetzen.

Vorzugsweise ist vorgesehen, dass nur ein vergleichsweise geringer Kraftaufwand erforderlich ist, wenn die Fixierungsmittel ein- oder ausgeklinkt werden. Dies kann durch die genannte Kugelrolle bzw. durch eine Kugel, Walze, Gleitstück oder Kufenstück oder dergleichen verwirklicht werden, die vorzugsweise so dimensioniert ist, dass sie in die Vertiefung einführbar ist, wenn die mobile Vorrichtung ihre gewünschte Endposition erreicht hat.

Denkbar ist es, diese Walze, Kugel bzw. Kugelrolle, Kufe oder Gleitstück an der Unterseite der mobilen Vorrichtung anzuordnen und in der Vertiefung der Führungszunge eingreifen zu lassen, wenn die mobile Vorrichtung ihre gewünschte Endposition erreicht hat.

In weiterer Ausgestaltung der Erfindung ist vorgesehen, dass das Gerät und/oder die mobile Vorrichtung einen oder mehrere Stützfüße und/oder eine oder mehrere Rollen aufweist.

In bevorzugter Ausgestaltung der Erfindung ist des weiteren vorgesehen, dass die Rollen, vorzugsweise die in Einschubrichtung vorne befindlichen Rollen oder auch alle Rollen der mobilen Vorrichtung flach und möglichst nicht oder wenig ausschwenkend konstruiert sind, damit ein Verhaken der Rollen mit dem Blutbehandlungsgerät möglichst vermieden wird. Vorzugsweise sind die Rollen bzw. Räder der mobilen Vorrichtung gemäß der DE 19 39 932 A1 ausgeführt, auf die insoweit Bezug genommen wird. Demnach können die Rollen bzw. Räder aus einem rotierbar angeordneten Nabenkörper bestehen, der auf seinem Umfang mehrere über den Nabenkörperumfang hinaus ragende drehbare Rollen aufweist, deren Drehachsen in Richtung der Tangenten an den Nabenkörperumfang verlaufen. Durch diese Ausgestaltung ist es möglich, die mobile Vorrichtung auch im belasteten Zustand ohne weiteres in zueinander senkrechten Richtungen zu bewegen.

Hinsichtlich weiterer denkbarer Ausgestaltungen der Rollen bzw. Räder wird auf die DE 19 39 932 A1 Bezug genommen.

Diese Bewegbarkeit (bidirektional oder multidirektional) der Rolle kann nur für die beim Einführen in die Aufnahme nachlaufenden, nur für die beim Einführen in die Aufnahme vorderen Rollen oder sowohl für die nachlaufenden als auch für die vorderen Rollen der mobilen Vorrichtung gelten.

Denkbar ist es, dass das Gerät an ihrer zu der offenen Seite der Aufnahme gewandten Seite einen oder mehrere Stützfüße und an der von der offenen Seite der Aufnahme abgewandten Seite eine oder mehrere Rollen aufweist.

Vorzugsweise ist vorgesehen, dass die Führungsmittel derart ausgebildet sind, dass die mobile Vorrichtung an ihrer zu dem Gerät gerichteten Seite zumindest am Anfang der Einführbewegung in die Aufnahme des Gerätes angehoben wird.

Dies führt dazu, dass die mobile Einheit leichter manövrierbar ist, was den Vorteil mit sich bringt, dass diese definiert und treffsicher in die Aufnahme eingefahren werden kann.

Weiterhin ist vorgesehen, dass das Gerät bei in die Aufnahme eingeführter mobiler Vorrichtung an ihrer zu der offenen Seite der Aufnahme gerichteten Seite angehoben ist.

Die vorliegende Erfindung betrifft des weiteren ein Verfahren zum Einführen einer mobilen Vorrichtung eines Systems gemäß einem der Ansprüche 1 bis 11 in ein Gerät eines Systems nach einem der Ansprüchen 1 bis 11, wobei die mobile Vorrichtung beim Einführen in die. Aufnahme des Gerätes an ihrer zu dem Gerät gewandten Seite angehoben wird, so dass das Gewicht der mobilen Vorrichtung auch von dem Gerät getragen wird. Dadurch wird die Wahrscheinlichkeit für ein Verrutschen des Gerätes vorzugsweise zu Beginn des Einführvorgangs durch Anpressen der Stützfüße oder Rollen an den Boden verringert, das heißt eine Bremswirkung wird ausgeübt, wenn die mobile Vorrichtung beispielsweise auf das Führungsmittel und/oder Fixierungsmittel des Gerätes einwirkt.

Die Erfindung betrifft des weiteren ein Verfahren zum Einführen einer mobilen Vorrichtung eines Systems gemäß einem der Ansprüche 1 bis 11 in ein Gerät eines Systems nach einem der Ansprüche 1 bis 11, wobei das Gerät beim Einführen der mobilen Vorrichtung in die Aufnahme des Gerätes eine Kippbewegung durchführt und nach dem Einführen der mobilen Vorrichtung in die Aufnahme des Gerätes an seiner zu der Einführseite der Aufnahme gewandten Seite angehoben ist.

Vorzugsweise ist das Gerät vor dem Einführen der mobilen Vorrichtung zu seiner Einführseite hin leicht geneigt.

Wird nun die mobile Vorrichtung eingeschoben, drückt diese bzw. deren Gleitrolle oder dergleichen mit fortschreitendem Einschieben zunehmend stärker auf das Gerät bzw. auf dessen Führungsmittel und/oder Fixierungsmittel, so dass das Gerät aus seiner geneigten Position in eine vorzugsweise gerade Position kippt. Dabei werden die vorderen, das heißt zur Einführseite geneigten Füße, insbesondere Standfüße des Gerätes angehoben. Das Gerät steht damit nur noch auf den beiden hinteren Füßen bzw. vorzugsweise auf den beiden hinteren Rollen.

Da das Gerät nach dem Einführen der mobilen Vorrichtung jedoch mit diesem eine Einheit bildet, ist das Gerät standsicher.

Wird die Gewichtskraft von Gerät und mobiler Vorrichtung in dem eingeschobenen Zustand nur noch von Rollen getragen, ist diese gesamte Einheit aus Gerät und mobiler Vorrichtung somit verschiebbar.

Weitere Einzelheiten und Vorteile der Erfindung werden anhand eines in der Zeichnung dargestellten Ausführungsbeispiels näher erläutert. Es zeigen:
- Figur 1:: eine perspektivische Ansicht eines Blutbehandlungsgerätes und einer mobilen Vorrichtung mit Tank vor dem Einführen der mobilen Vorrichtung,
- Figur 2:: eine schematische Seitenansicht des unteren Bereiches des Blutbehandlungsgerätes vor Einführen der mobilen Vorrichtung,
- Figur 3:: eine schematische Seitenansicht des unteren Bereiches des Blutbehandlungsgerätes mit eingeführter mobiler Vorrichtung,
- Figur 4:: eine perspektivische Darstellung der Führungszunge des Blutbehandlungsgerätes und
- Figur 5:: eine perspektivische Ansicht eines Blutbehandlungsgerätes und einer mobilen Vorrichtung ohne Tank vor dem Einführen der mobilen Vorrichtung.

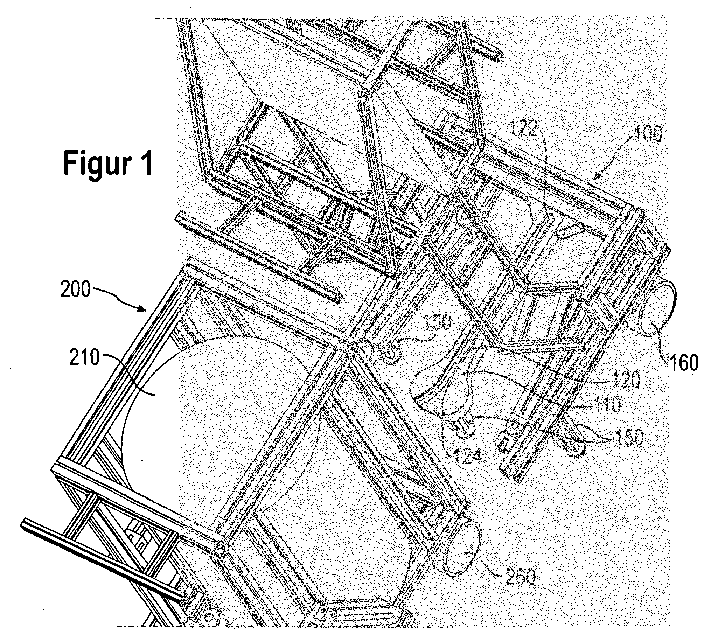

In Figur 1 ist mit dem Bezugszeichen 100 ein Blutbehandlungsgerät gekennzeichnet, bei dem es sich um ein Dialysegerät 100 handelt, das in der Zeichnung nicht näher dargestellte Mittel zur Durchführung einer Blutbehandlung, insbesondere einer Dialysebehandlung aufweist. Diese Mittel können beispielsweise einen extrakorporalen Blutkreislauf bzw. die dazu erforderlichen Schläuche, Pumpen, wenigstens einen Dialysator, etc. aufweisen.

Wie dies aus Figur 1 ersichtlich ist, weist das Blutbehandlungsgerät 100 einen einseitig offenen U-förmigen Rahmen auf, wobei die offene Seite des Rahmens die Einführseite zum Einführen der mobilen Vorrichtung 200 darstellt. Die Aufnahme des Gerätes 100 weist eine Breite auf, so dass die mobile Vorrichtung 200 in die Aufnahme eingeschoben werden kann. Die mobile Vorrichtung 200 weist einen Tank 210 auf, in dem Behandlungsflüssigkeit zur Durchführung der Behandlung, insbesondere eine Dialysierflüssigkeit aufgenommen ist.

Mit dem Bezugszeichen 160 sind im rückwärtigen Bereich des Blutbehandlungsgerätes 100 angeordnete Bockrollen gekennzeichnet. Mit dem Bezugszeichen 150 sind Standfüße gekennzeichnet, die sich in dem Bereich des Blutbehandlungsgerätes 100 erstrecken, der zur Einführseite hin gerichtet ist. Wie dies aus Figur 1 ersichtlich ist, erstreckt sich einer der Standfüße 150 auf der Unterseite einer Führungszunge 110, die sich in Einführrichtung und damit senkrecht zu der Einführebene bzw. senkrecht zu der offenen Seite des Blutbehandlungsgerätes 100 erstreckt. Mit dem Bezugszeichen 260 sind Bockrollen der mobilen Vorrichtung 200 dargestellt. Diese können lenkbar bzw. bi- oder multidirektional rollend oder auch starr und nur in eine Richtung rollend ausgeführt sein. Auf die obigen Ausführungen zu der DE 19 39 932 A1 wird entsprechend Bezug genommen.

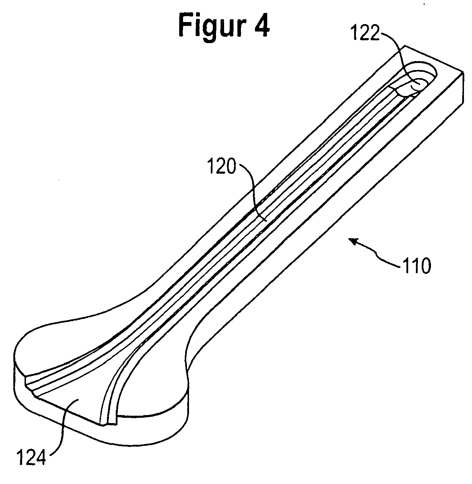

Wie dies insbesondere aus Figur 4 hervorgeht, weist die Führungszunge 110 eine Nut 120 auf, die sich in Einführrichtung erstreckt und die eine vergleichsweise breite Einführöffnung 124 aufweist.

Die Führungszunge 110 ist frei auskragend ausgeführt und weist vorzugsweise an ihrem freien Ende eine Abrundung auf.

Im Bereich des hinteren Nutendes befindet sich eine gegenüber dem Nutgrund nach unten zurückversetzte Vertiefung 122, die zur Aufnahme einer aus Figur 1 nicht ersichtlichen Kugel bzw. Kugelrolle der mobilen Vorrichtung 200 dient.

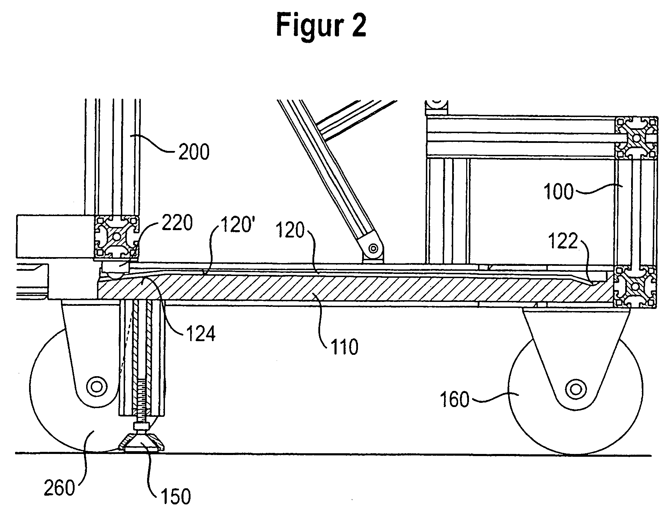

Figur 2 zeigt in einer schematischen Ansicht eine Seitendarstellung. Aus dieser Darstellung ist erkennbar, dass die Nut 120 einen Nutgrund 120' aufweist, der im Bereich der links dargestellten Einführseite leicht ansteigt und der in seinem hinteren rechts dargestellten Ende in die genannte Vertiefung 122 übergeht.

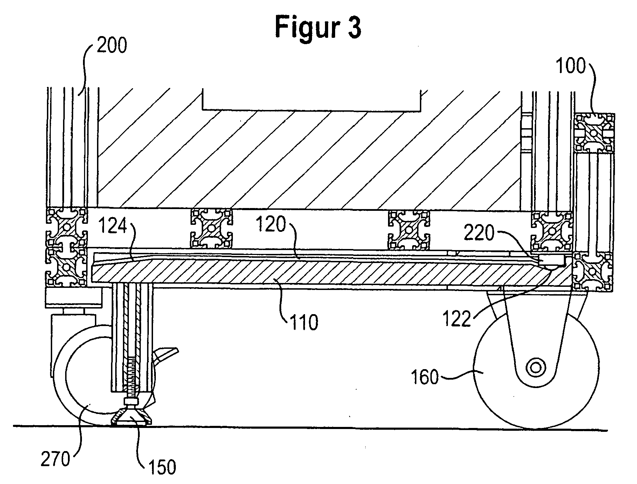

Mit dem Bezugszeichen 220 ist eine Kugelrolle gekennzeichnet, die an der mobilen Vorrichtung 200 derart angeordnet ist, dass sie in die Nut 120 eingeführt werden kann und in dem vollständig eingeführten Zustand in der Vertiefung 122 angeordnet ist, wie dies aus Figur 3 ersichtlich ist. Aus Figur 3 ist weiter ersichtlich, dass die mobile Vorrichtung 200 an ihrem in Einführrichtung nachlaufenden Ende ebenfalls Rollen 270 aufweist. Diese sind vorzugsweise schwenkbar und/oder bi- oder multidirektional rollend ausgeführt, wie dies oben näher, insbesondere Bezug nehmend auf die DE 19 39 932 A1, beschreiben wurde.

Die Führungszunge 110 kann beispielsweise als Frästeil ausgelegt sein, aber auch anderweitig ausgeführt sein. In dem hier dargestellten Ausführungsbeispiel ist sie das zentrale, mittig in der Aufnahme befindliche Führungselement und gewährleistet eine definierte Andockfahrt der mobilen Vorrichtung 200 in die Aufnahme des Blutbehandlungsgerätes 100 bzw. in die Aufnahme eines Befüllgeräts oder eines Entleergerätes, die in den Zeichnungen nicht dargestellt sind.

Die Führungszunge 110 ist an einer der Gerätekomponenten, in dem Ausführungsbeispiel an dem Blutbehandlungsgerät 100 fest montiert.

Wie aus Figur 4 ersichtlich, weist die Führungszunge 110 eine Führungsgeometrie auf, die eine Interaktion mit der Kugelrolle 220 ermöglicht.

Die Kugelrolle 220 ist fester Bestandteil der weiteren Gerätekomponente, in dem vorliegenden Ausführungsbeispiel ist sie fester Bestandteil der mobilen Vorrichtung 200.

Wie oben ausgeführt und wie in Figur 1 ersichtlich, weist die Führungszunge 110 eine vergleichsweise breite Öffnung 124 der Nut auf. Diese breite Öffnung 124 gewährleistet, dass die Kugelrolle 220 sicher auf die Bahn bzw. in die Nut 120 der Führungszunge 110 aufgefahren werden kann.

Figur 2 zeigt den Beginn des Andockens. Zunächst trifft die Kugelrolle 220 auf die Öffnung 124 der Nut 120 der Führungszunge 110. Das Blutbehandlungsgerät 100 bzw. die Befülleinheit oder Entleereinheit befindet sich in diesem Zustand, das heißt am Anfang der Andockfahrt in leicht nach vorne, d. h. zur mobilen Vorrichtung 200 hin gekipptem Zustand.

Dies führt dazu, dass das Höhenniveau der Führungszunge 110 im Bereich ihrer Öffnung 124 unter dem Niveau der Kugelrolle 220 liegt. Mit zunehmender Einschubbewegung erhöht sich das Niveau der Nut 120 bzw. der Führungsbahn, was dazu führt, dass die Bockrollen 260 an der in Figur 2 dargestellten Hinterseite der mobilen Vorrichtung 200 vom Boden abgehoben werden.

Dies führt dazu, dass die mobile Vorrichtung 200 nur noch mit der Kugelrolle 220 und der in Figur 2 nicht dargestellten vorzugsweise eine Schwenkbewegung bzw. bi- oder multidirektionale Bewegung ermöglichende Vorderachse bzw. der sonstigen Stützelemente fahrbar ist und dadurch genug Fahrfreiheiten besitzt, um definiert und Treffsicher in das Gerät 100 eingefahren zu werden.

Ein weiterer wesentlicher Vorteil des Abhebens der mobilen Vorrichtung 200 an der zu dem Gerät 100 gerichteten Seite besteht darin, dass das Gewicht der mobilen Vorrichtung 200 teilweise von dem Gerät 100 übernommen wird, was dazu führt, dass eine Bremswirkung über den Stützfuß 150 ausgeübt wird, der sich an der Führungszunge 110 erstreckt.

Figur 3 zeigt das Ende der Andockfahrt, bei dem sich die mobile Vorrichtung 200 vollständig in der Aufnahme befindet.

Wie aus Figur 3 ersichtlich, fährt am Ende der Andockfahrt die Kugelrolle 220 über eine Rampe in eine Vertiefung 122 herunter und wird in der Vertiefung 122 aufgenommen. Dabei werden die Stützfüße 150 des Gerätes 100 vom Boden abgehoben, da das Gerät 100 leicht nach hinten gekippt wird. Dies führt dazu, dass die Rolle 260 der mobilen Vorrichtung 200 nun wieder am Boden aufsitzt, da die Niveaus beider Komponenten (Gerät und mobile Vorrichtung) am Ende durch die Rampe der Führungsnut 120 angepaßt werden.

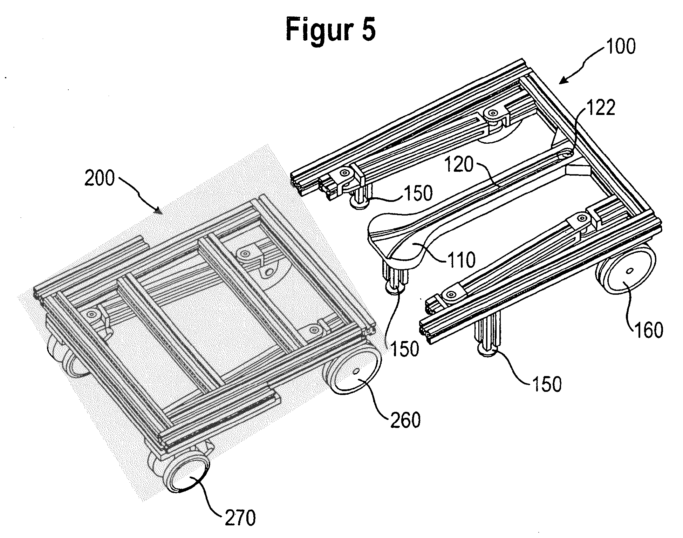

Figur 5 zeigt eine der Anordnung gemäß Figur 1 vergleichbare Ausführung der vorliegenden Erfindung. Die Anordnungen gemäß Figur 1 und Figur 5 unterscheiden sich dadurch, dass die mobile Vorrichtung 200 gemäß Figur 5 keinen Tank aufweist.

Wie dies weiter aus Figur 5 hervorgeht, weist die mobile Vorrichtung 200 nicht nur an ihrer vorlaufenden, zum Dialysegerät 100 gerichteten Seite, sondern auch auf ihrer nachlaufenden Seite Rollen 260, 260' auf.

Darüber hinaus sind gleiche oder funktionsgleiche Teile gemäß Figur 5 mit denselben Bezugszeichen gekennzeichnet, wie in Figur 1. Wie dies weiter aus Figur 5 hervorgeht, steht die mobile Vorrichtung 200 gemäß diesem Ausführungsbeispiel nur auf Rollen 260, 260', wohingegen das Dialysegerät 100, das in Figur 5 nur im Grundaufbau dargestellt ist, nur auf der von der Einführöffnung abgewandten Seite, das heißt dem hinteren Bereich über Rollen 160 und ansonsten über Stützfüße 150 verfügt. Eine entsprechende Ausführung und Anordnung von Rollen und Stützfüßen ist auch für die weiteren Ausführungsformen der Erfindung denkbar und nicht auf die Anordnung in Figur 5 beschränkt.

## Patentansprüche

1. System mit wenigstens einem Blutbehandlungsgerät (100), insbesondere Dialysegerät (100), mit Mitteln zur Durchführung einer Blutbehandlung, insbesondere einer Dialysebehandlung, oder mit wenigstens einem Gerät mit Mitteln zur Befüllung oder Entleerung einer mobilen Vorrichtung (200) mit einer bzw. von einer Behandlungsflüssigkeit, insbesondere Dialysierflüssigkeit, und mit der mobilen Vorrichtung (200) mit wenigstens einem Tank (210), in dem sich eine Behandlungsflüssigkeit, insbesondere Dialysierflüssigkeit, befindet oder aufnehmbar ist, zur Versorgung des genannten Blutbehandlungsgerätes (100) mit der Behandlungsflüssigkeit während einer durch das Blutbehandlungsgerät (100) durchgeführten Blutbehandlung, oder zur Verbindung mit dem genannten Gerät,
wobei das Gerät (100) wenigstens eine Aufnahme aufweist, die derart ausgebildet ist, dass in der Aufnahme die mobile Vorrichtung (200) aufnehmbar ist, wobei das Gerät (100) und die mobile Vorrichtung Führungsmittel und vorzugsweise Fixierungsmittel aufweist, die derart ausgeführt sind, dass die mobile Vorrichtung (200) mit Hilfe der Führungsmittel und vorzugsweise der Fixierungsmittel in die Aufnahme einführbar und/oder in der Aufnahme fixierbar ist,
**dadurch gekennzeichnet,**
**dass** die Führungsmittel wenigstens eine Führungsbahn, deren Niveau sich mit zunehmender Einschubbewegung erhöht und die wenigstens eine Vertiefung (122) aufweist, und wenigstens eine Kugel oder Walze oder eine Kufe oder ein Gleitstück umfassen, die in oder auf der Führungsbahn laufen und zumindest teilweise in der Vertiefung (122) aufnehmbar sind, und mit fortschreitendem Einschieben der mobilen Vorrichtung zunehmend stärker auf die Führungsbahn drücken, so dass das Gerät (100) bei in die Aufnahme des Gerätes (100) eingeführter mobiler Vorrichtung (200) an seiner zu der offenen Seite der Aufnahme gerichteten Seite angehoben ist.

2. System nach Anspruch 1, **dadurch gekennzeichnet, dass** die Führungsmittel und/oder die Fixierungsmittel wenigstens eine Führungszunge (110) und/oder wenigstens eine Führungsschiene umfassen.

3. System nach Anspruch 1 oder 2, **dadurch gekennzeichnet, dass** die Führungsmittel und/oder die Fixierungsmittel wenigstens eine Nut (120) oder die Führungsbahn umfassen.

4. System nach Anspruch 2 und 3, **dadurch gekennzeichnet, dass** die Nut (120) oder Führungsbahn in der Führungszunge (110), vorzugsweise auf der Oberseite der Führungszunge (110) angeordnet ist.

5. System nach einem der vorhergehenden Ansprüche, **dadurch gekennzeichnet, dass** die Vertiefung (122) am Nutende oder am Ende der Führungsbahn angeordnet ist.

6. System nach einem der Ansprüche 3 bis 5, **dadurch gekennzeichnet, dass** die Nut (120) oder die Führungsbahn wenigstens eine Einführöffnung (124) aufweist, die eine größere Breite aufweist, als der sich an die Einführöffnung anschließende Teil der Nut (120) oder der Führungsbahn.

7. System nach einem der Ansprüche 2 bis 6, **dadurch gekennzeichnet, dass** eine Einführrichtung besteht, in der die mobile Vorrichtung (200) in die Aufnahme des Gerätes (100) einführbar ist und dass sich die Führungszunge (110) in Einführrichtung erstreckt.

8. System nach einem der vorhergehenden Ansprüche, **dadurch gekennzeichnet, dass** die Fixierungsmittel Mittel zur formschlüssigen Fixierung, insbesondere Rastmittel umfassen oder aus diesen bestehen.

9. System nach einem der vorhergehenden Ansprüche, **dadurch kennzeichnet, dass** das Gerät (100) und/oder die mobile Vorrichtung (200) einen oder mehrere Stützfüße (150) und/oder eine oder mehrere Rollen (160, 260, 270) aufweist.

10. System nach Anspruch 9, **dadurch gekennzeichnet, dass** das Gerät (100) an seiner zu der offenen Seite der Aufnahme gewandten Seite einen oder mehrere Stützfüße (150) und an seiner von der offenen Seite der Aufnahme abgewandten Seite eine oder mehrere Rollen (160) aufweist, und/oder dass die mobile Vorrichtung (200) keine Stützfüße, sondern Rollen (260, 270) aufweist.

11. System nach einem der vorhergehenden Ansprüche, **dadurch gekennzeichnet, dass** die Führungsmittel derart ausgebildet sind, dass die mobile Vorrichtung (200) an ihrer zu dem Gerät (100) gerichteten Seite bei der Einführung in die Aufnahme des Gerätes (100) angehoben wird.

12. Verfahren zum Einführen einer mobilen Vorrichtung (200) eines Systems gemäß einem der Ansprüche 1 bis 11 in ein Gerät (100) eines Systems nach einem der Ansprüche 1 bis 11, **dadurch gekennzeichnet, dass** das Gerät (100) beim Einführen der mobilen Vorrichtung (200) in die Aufnahme des Gerätes (100) eine Kippbewegung durchführt und nach dem Einführen der mobilen Vorrichtung (200) in die Aufnahme des Gerätes (100) an seiner zu der Einführseite der Aufnahme gewandten Seite angehoben ist.

13. Verfahren nach Anspruch 12, **dadurch gekennzeichnet, dass** die mobile Vorrichtung (200) beim Einführen in die Aufnahme des Gerätes (100) an ihrer zu dem Gerät (100) gewandten Seite angehoben wird, so dass das Gewicht der mobilen Vorrichtung (200) auch von dem Gerät (100) getragen wird.

## Claims

1. A system having at least one blood treatment device (100), in particular a dialysis device (100), having means for the carrying out of a blood treatment, in particular a dialysis treatment, or having at least one device having means for the filling of a mobile apparatus (200) with or for the emptying of a mobile apparatus (200) of a treatment fluid, in particular a dialysis fluid, and having the mobile apparatus (200) having at least one tank (210) in which a treatment fluid, in particular a dialysis fluid, is present or can be received for the supply of said blood treatment device (100) with the treatment fluid during a blood treatment carried out by the blood treatment device (100) or for the connection to said device,
wherein the device (100) has at least one mount which is made such that the mobile apparatus (200) can be received in the mount, with the device (100) and the mobile apparatus having guide means and preferably fixing means which are designed such that the mobile apparatus (200) can be introduced into the mount and/or fixed in the mount with the aid of the guide means and preferably of the fixing means,
**characterized in that**
the guide means comprise at least one guide track which rises in level with an increasing insertion movement and which has at least one recess (122) and at least one ball or roll or a blade or a slide piece which run in or on the guide track and can be received at least partly in the recess (122) and press increasingly strongly against the guide track with progressing insertion of the mobile apparatus so that, with the mobile apparatus (200) inserted into the mount of the device (100), the device (100) is raised at its side facing the open side of the mount.

2. A system in accordance with claim 1, **characterized in that** the guide means and/or the fixing means comprise at least one guide tongue (110) and/or at least one guide rail.

3. A system in accordance with either of claims 1 and 2, **characterized in that** the guide means and/or the fixing means comprise at least one groove (120) or the guide track.

4. A system in accordance with either of claims 2 and 3, **characterized in that** the groove (120) or guide track is arranged in the guide tongue (110), preferably on the upper side of the guide tongue (110).

5. A system in accordance with one of the preceding claims, **characterized in that** the recess (122) is arranged at the groove end or at the end of the guide track.

6. A system in accordance with one of the claims 3 to 5, **characterized in that** the groove (120) or the guide track has at least one introduction opening (124) which has a larger width than the part of the groove (120) or of the guide track adjoining the introduction opening.

7. A system in accordance with one of the claims 2 to 6, **characterized in that** an introduction direction is present in which the mobile apparatus (200) can be introduced into the mount of the device (100); and **in that** the guide tongue (110) extends in the introduction direction.

8. A system in accordance with one of the preceding claims, **characterized in that** the fixing means include means for the shaped matched fixing, in particular comprise or consist of latch means.

9. A system in accordance with one of the preceding claims, **characterized in that** the device (100) and/or the mobile apparatus (200) has one or more support feet (150) and/or one or more rollers (160, 260, 270).

10. A system in accordance with claim 9, **characterized in that** the device (100) has one or more support feet (150) at its side facing the open side of the mount and one or more rollers (160) at its side remote from the open side of the mount; and/or **in that** the mobile apparatus (200) does not have any support feet, but rollers (260, 270).

11. A system in accordance with one of the preceding claims, **characterized in that** the guide means are made such that the mobile apparatus (200) is raised at its side directed toward the device (100) on the introduction into the mount of the device (100).

12. A method for the introduction of a mobile apparatus (200) of a system in accordance with one of the claims 1 to 11 into a device (100) of a system in accordance with one of the claims 1 to 11, **characterized in that** the device (100) carries out a tilting movement on the introduction of the mobile apparatus (200) into the mount of the device (100) and is raised at its side facing the introduction side of the mount after the introduction of the mobile apparatus (200) into the mount of the device (100).

13. A method in accordance with claim 12, **characterized in that** the mobile apparatus (200) is raised at its side facing the device (100) on the introduction into the mount of the device (100) so that the weight of the mobile apparatus (200) is also carried by the device (100).

## Revendications

1. Système comprenant au moins un appareil de traitement du sang (100), en particulier un appareil de dialyse (100), qui comprend des moyens destinés à exécuter un traitement du sang, en particulier un traitement par dialyse, ou comprenant au moins un appareil doté de moyens destinés à remplir ou vider un dispositif mobile (200) d'un fluide de traitement, en particulier un fluide dialyseur, et comprenant le dispositif mobile (200) doté d'au moins un réservoir (210), dans lequel un fluide de traitement, en particulier un fluide dialyseur, se trouve ou peut être reçu, pour alimenter ledit appareil de traitement du sang (100) avec le fluide de traitement pendant un traitement du sang exécuté par l'appareil de traitement du sang (100), ou pour la liaison avec ledit appareil,
l'appareil (100) comportant au moins un logement, qui est formé de telle manière que le dispositif mobile (200) peut être reçu dans le logement,
l'appareil (100) et le dispositif mobile comportant des moyens de guidage et de préférence des moyens de fixation, qui sont réalisés de telle manière que le dispositif mobile (200) peut être introduit ou fixé dans le logement à l'aide des moyens de guidage et de préférence des moyens de fixation,
**caractérisé en ce que**
les moyens de guidage comportent au moins une voie de guidage, dont le niveau s'élève au fur et à mesure que le mouvement de poussée progresse et qui comporte au moins un enfoncement (122), et comprennent au moins une bille ou un cylindre ou un patin ou une pièce coulissante, qui se déplacent dans ou sur la voie de guidage et peuvent être reçus au moins en partie dans l'enfoncement (122), et qui appuient de plus en plus fort sur la voie de guidage au fur et à mesure que le dispositif mobile est enfoncé, de telle manière que l'appareil (100) est soulevé sur son côté orienté vers le côté ouvert de l'enfoncement lorsque le dispositif mobile (200) est introduit dans le logement de l'appareil (100).

2. Système selon la revendication 1, **caractérisé en ce que** les moyens de guidage et/ou les moyens de fixation comprennent au moins une languette de guidage (110) et/ou au moins un rail de guidage.

3. Système selon la revendication 1 ou 2, **caractérisé en ce que** les moyens de guidage et/ou les moyens de fixation comprennent au moins une rainure (120) ou la voie de guidage.

4. Système selon les revendications 2 et 3, **caractérisé en ce que** la rainure (120) ou voie de guidage est disposée dans la languette de guidage (110), de préférence sur le côté supérieur de la languette de guidage (110),

5. Système selon l'une des revendications précédentes, **caractérisé en ce que** l'enfoncement (122) est disposé à l'extrémité de la rainure ou à l'extrémité de la voie de guidage.

6. Système selon l'une des revendications 3 à 5, **caractérisé en ce que** la rainure (120) ou la voie de guidage comporte au moins une ouverture d'introduction (124) qui présente une largeur supérieure à la partie de la rainure (120) ou de la voie de guidage adjacente à l'ouverture d'introduction.

7. Système selon l'une des revendications 2 à 6, **caractérisé en ce qu'**il y a une direction d'introduction, dans laquelle le dispositif mobile (200) peut être introduit dans le logement de l'appareil (100) et **en ce que** la languette de guidage (110) s'étend dans la direction d'introduction.

8. Système selon l'une des revendications précédentes, **caractérisé en ce que** les moyens de fixation comprennent des moyens destinés à une fixation par complémentarité de formes, en particulier des moyens d'encliquetage, ou sont composés de ceux-ci.

9. Système selon l'une des revendications précédentes, **caractérisé en ce que** l'appareil (100) et/ou le dispositif mobile (200) comporte un ou plusieurs pieds d'appui (150) et/ou une ou plusieurs roulettes (160, 260, 270).

10. Système selon la revendication 9, **caractérisé en ce que** l'appareil (100) comporte un ou plusieurs pieds d'appui (150) sur son côté tourné vers le côté ouvert du logement et une ou plusieurs roulettes (160) sur son côté opposé au côté ouvert du logement, et/ou **en ce que** le dispositif mobile (200) ne comporte pas de pieds d'appui, mais des roulettes (260, 270).

11. Système selon l'une des revendications précédentes, **caractérisé en ce que** les moyens de guidage sont formés de telle manière que le dispositif mobile (200) est soulevé sur son côté orienté vers l'appareil (100) lors de l'introduction dans le logement de l'appareil (100).

12. Procédé d'introduction d'un dispositif mobile (200) d'un système selon l'une des revendications 1 à 11 dans un appareil (100) d'un système selon l'une des revendications 1 à 11, **caractérisé en ce que** l'appareil (100) exécute un mouvement de basculement lors de l'introduction du dispositif mobile (200) dans le logement de l'appareil (100) et est soulevé sur son côté tourné vers le côté d'introduction du logement après l'introduction du dispositif mobile (200) dans le logement de l'appareil (100).

13. Procédé selon la revendication 12, **caractérisé en ce que** le dispositif mobile (200) est soulevé sur son côté tourné vers l'appareil (100) lors de l'introduction dans le logement de l'appareil (100), de telle manière que le poids du dispositif mobile (200) soit porté aussi par l'appareil (100).
